# EUROPEAN PATENT APPLICATION

(11) **EP 1 016 392 A1**
(43) Date of publication of application: **05.07.2000**
(21) Application number: 98124828.9
(22) Date of filing: 30.12.1998
(51) Int. Cl.: A61F 13/15

(54) **Body applied tridimensional disposable article having a panty fastening system with time delayed activation**

(71) Applicant: THE PROCTER & GAMBLE COMPANY, Cincinnati, Ohio 45202 (US)
(72) Inventor: Wierlacher, Stefan, 65122 Pescara (IT); Nepa, Lucrezia, 66100 Chieti (IT)
(74) Representative: Hirsch, Uwe Thomas M.H.

(57) **Abstract**

A tridimensional disposable absorbent article having a tridimensional shape prior to use with a generally upwardly convex rear region, having a body facing surface and a garment facing surface, a longitudinal symmetry plane, a front end edge and a rear end edge, and comprising a liquid pervious topsheet, a backsheet joined to said topsheet and an absorbent core intermediate the backsheet and the topsheet. The absorbent article is intended for direct application to the user's body before an undergarment is worn, and comprises a system for attachment of the article to the undergarment with an adhesive means that can be activated after the undergarment has been worn.

## Description

### FIELD OF THE INVENTION

The present invention relates to disposable absorbent articles. Disposable absorbent articles are considered to be absorbent devices designed to be worn externally of the body by a user and to receive fluids discharged from the body. In particular the present invention relates to disposable absorbent sanitary napkins, catamenials, incontinence inserts, and pantiliners that are capable of providing enhanced fit for the body and reduced leakage by having a tridimensional shape prior to use that matches the non-planar surfaces and the non-linear grooves of the body. The tridimensional disposable absorbent articles of the present invention are preferably applied directly to the user's body, and comprise a panty fastening system for attachment of the article to the undergarment after start of wearing of said article.

### BACKGROUND OF THE INVENTION

In their basic form, disposable absorbent articles comprise an absorbent core interposed between a pervious body-contacting element (alternatively referred to as a topsheet or an overwrap) and an impervious protective barrier (alternatively referred to as a backsheet). The absorbent element is, of course, intended to receive and contain the fluids discharged from the body. The body-contacting element is intended to provide comfortable and dry-feeling contact with body surfaces while allowing free passage of fluids therethrough into the absorbent element. The protective barrier is intended to prevent the fluids which are expelled or which escape from the absorbent element from soiling the user's garments.

Major disadvantages of known disposable absorbent articles intended to be worn externally of the body, e.g. leakage, wet/dirty feeling, discomfort, are related to the poor body fit achieved by these articles that are either substantially flat prior to use and must then be squeezed or folded into the right shape to follow the body surface, or, alternatively, are shaped before use, but still need improvement in order to get a better fit with the complex shapes of the users anatomy. Moreover, most known disposable absorbent articles are intended to be applied to the panty, and typically fixed to it by an adhesive, before wearing the panty with the applied absorbent article, and this does not facilitate a good fit with the body anatomy, also owing to differences in wearing habits and in panty styles.

With respect to e.g. disposable sanitary napkins several attempts have been made in the art to improve body contact with the wearer, and hence to absorb fluids upon discharge and thereby minimise soiling, by means of a sanitary napkin having an anatomically shaped configuration with the capability of conforming to the body anatomy.

On female users this type of sanitary napkins attempts to contact and absorb menses immediately as it leaves the vestibule.

Body conforming sanitary napkins are known in the art, both those that are flat prior to use, and that are intended to shape or mould in use to match the wearer's anatomy, and those that are shaped prior to use. In European Patent Application EP 97110735.4 filed on 1 July 1997 disposable absorbent articles, particularly sanitary napkins, have been described, which are provided before use with an improved tridimensional structure capable of conforming to the various complex body shapes of the female anatomy comprising non-linear grooves and non-planar surfaces, in order to provide increased body fit and comfort, and reduced leakage.

Such disposable absorbent articles comprise a longitudinally oriented ridge in the central and rear portions having a slope that decreases rearwardly, and are preferably intended for direct application to the users body.

In European patent application No. 97122739.2 filed on 23 December 1997, entitled "Tridimensional disposable absorbent article having a slit in the rear region" (P&G case CM1667Q), a tridimensional disposable absorbent article similar to that disclosed in the above mentioned application EP 97110735.4 is described. The absorbent article has a tridimensional shape prior to use and preferably comprises an upwardly concave front region and an upwardly convex rear region preferably having an inverted V shaped longitudinal ridge. The absorbent article of EP 97122739.2 also comprises a slit or cut in the rear region which extends from the rear end edge and is oriented substantially longitudinally. The rear cut or slit gives the preferably inverted V shaped rear portion of the absorbent article the capability of bending around an axis which is perpendicular to the symmetry plane of the article, in order to better fit the various body shapes, specifically in the area of the gluteal groove, where the rear region of the absorbent article preferably extends in order to provide a better protection.

The tridimensional disposable absorbent articles described in EP application No. 97122739.2 has the capability of better adjusting its shape, particularly its upwardly convex rear region, to the different anatomies taking into account the possible interactions with the undergarment, and any variations experienced during the wearing time, due e.g. to the wearer's movements, at the same time providing a better fit and a proper positioning of the rear region interested by the rear slit or cut with respect to the preferred acquisition region of the absorbent article

Preferably, the three dimensional absorbent articles of EP applications No. 97110735.4 and No. 97122739.2 also have a structural tridimensionality, by "structural tridimensionality" being meant that the three dimensional structure cannot be completely flattened, i.e., spread out without portions still folded on themselves, onto a flat surface while keeping its integrity, that is, without being in any case e.g. torn, crushed or squeezed.

Disposable absorbent articles having a three dimensional shape prior to use, preferably with the structural tridimensionality, such as those described in the above mentioned applications EP 97110735.4 and EP 97122739.2, do provide a good fit to the anatomy, and therefore a better comfort to the user and a reduced leakage.

Preferably the tridimensional disposable absorbent articles of the two European patent applications mentioned above are intended to be applied directly to the user's body before an undergarment such as a panty is worn, rather than being applied first to the panty prior to wearing the panty itself with the absorbent article attached thereon.

Direct application to the body of the tridimensional disposable absorbent articles of the above mentioned European applications is in fact greatly beneficial in terms of body fit and of protection provided by the article. Owing to its structure and to its capability to conform and fit the anatomy of the user a tridimensional disposable absorbent article of the types described in EP 97110735.4 and EP 97122739.2 is capable to stay in place during the use also without any additional means intended to fasten the article to an undergarment.

However, the provision of a panty fastening means in a tridimensional disposable absorbent article intended for direct application to the users body is also desirable, in order to attach the article to the undergarment after the article has been applied to the body. Disposable absorbent articles are usually not meant to be disposed of every time the user visits the toilet e.g. to urinate or to check the condition of the article itself. Therefore they have to be securely stored during the check/urination, and then reapplied to the body, and this is of course true also for tridimensional disposable absorbent articles that are applied directly to the body.

If such a pad, after direct application to the body, is not attached to the panty, the user has in fact to handle it directly in some way during check/urination, typically by directly removing it from the body and then holding it, to avoid the risk that the possibly soiled pad falls off the body after panty pull down. In any case, even when the article can be handled safely by the user, there is the further need for the user of a clean and convenient place where to put it in order to have both hands free. The article has to be then reapplied directly to the body, if no change is necessary.

Attachment of the tridimensional absorbent article to the undergarment would facilitate the entire procedure, since the article is removed from the body by simply pulling down the panty with the article attached thereto, can be subsequently kept onto the panty during check/urination, and in case of subsequent reapplication, this can also be simply done via the panty. In case of reapplication in fact the right positioning of the article with respect to both the panty and the body, achieved through the first application directly to the body, and subsequent wearing of the panty, is in fact kept.

Traditional panty fastening systems are however not particularly suitable for tridimensional disposable absorbent articles that are to be applied directly to the users body prior to wearing the undergarment, in order to achieve the preferred improved body fit. Such panty fastening systems in fact typically consist of a layer of a pressure sensitive adhesive applied in various patterns onto most of the garment facing surface of the backsheet of a disposable absorbent article, and usually protected before use by a release paper. In a tridimensional disposable absorbent article applied directly to the body this adhesive layer has to be exposed preferably after application, but necessarily before the panty is pulled up. When the panty is pulled up it contacts the adhesive of the panty fastening system, and immediately adheres to it, at least to a certain extent. This contact and adhesion are not achieved in the best conditions since the panty is typically in a stretched condition when it is being pulled up, and a correct positioning of the panty, with a minimum and relatively constant level of stress and stretching in the panty structure, usually occurs after contact and adhesion with the panty fastening adhesive have already been achieved. This leads to the development of tensions and forces exerted by the panty onto the tridimensional absorbent article, due to relative movements between them, also possibly increased by users activity during the wearing time, and the article can therefore be moved from its proper position. Possible adjustments of the position of the panty relative to the article after panty pull up are neither simple nor very effective owing to the adhesion immediately achieved between the absorbent article and the panty.

These negative effects are further increased by the extension of the area covered by the panty fastening adhesive, which usually comprises most of the garment facing surface of the backsheet of the disposable absorbent article, at least in longitudinal direction.

It is therefore an object of the present invention to provide a tridimensional disposable absorbent article that is intended for direct application onto the user's body, and also comprises a panty fastening system which overcomes the drawbacks described above by allowing an attachment of the absorbent article to the undergarment which can be achieved independently of the panty pull up, i.e., which is activated after the panty has been actually worn.

It is a further object of the present invention to provide such a disposable absorbent article where the panty fastening system further provides a secure attachment of the article to the undergarment which also allows relative movements between the close body fitting disposable absorbent article and the undergarment during the wearing time, and through the user's activity.

### SUMMARY OF THE INVENTION

The present invention refers to tridimensional disposable absorbent articles for wearing against a user's body and intended for direct application to the user's body before an undergarment is worn. The tridimensional disposable absorbent article has a tridimensional shape prior to use and also has a body facing surface and a garment facing surface, a front region and a rear region, a central region comprised between the front region and the rear region, and a longitudinal symmetry plane. The tridimensional disposable absorbent article also comprises a liquid pervious topsheet, a liquid impervious backsheet joined to the topsheet, and an absorbent core intermediate the topsheet and the backsheet. The tridimensional disposable absorbent article further comprises on the garment facing surface of the backsheet a panty fastening system for attachment of the article to an undergarment during the use of the article. The panty fastening system comprises an adhesive means having a time delayed activation, such that the activation is achieved after the undergarment has been worn.

### BRIEF DESCRIPTION OF THE DRAWINGS

While the specification concludes with claims particularly pointing out and distinctly claiming the present invention, it is believed that the present invention will be better understood from the following description in conjunction with the following drawings:
FIG. 1 is a perspective view of one embodiment of a sanitary napkin according to the present invention, seen from the side thereof that faces the wearer in use;
FIG. 2 is a cross-sectional view of the sanitary napkin of FIG. 1 on line 2-2;
FIG. 3 is a top view of the sanitary napkin of FIG. 1;
FIG. 4 is a curve taken from an anatomical section of the body of a wearer which schematically represents the central non linear groove of the female anatomy as seen in lateral direction;
FIGS. 5a, 5b, and 5c are cross-sectional views of the sanitary napkin of FIG. 1 on lines 5a-5a, 5b-5b, and 5c-5c, respectively;
FIG. 6 is a perspective view of the sanitary napkin of FIG. 1, seen from the side that lies remote from the wearer in use;
FIGS. 7a and 7b are perspective views of a sanitary napkin according to the present invention similar to that illustrated in FIGS. 1 to 6, showing two in use positions of a different embodiment of the present invention.
FIGS. 8a, 8b, 8c, and 8d are plan views of sanitary napkins similar to those of FIG. 1, seen from the side that lies remote from the wearer in use, and showing different embodiments of the panty fastening system of the present invention.

### DETAILED DESCRIPTION OF THE INVENTION

This invention relates to tridimensional disposable absorbent articles having a tridimensional shape prior to use, and preferably having an upwardly convex, more preferably transversely inverted V shaped rear region, which exhibit absorbency for bodily fluids, the protection of the user's garments from soiling, and improved physical comfort to the user, which are also easy to produce and to package. Such articles are preferably provided in said upwardly convex rear region with at least a cut or a slit extending from the rear end edge substantially in longitudinal direction. Said at least one cut or slit defines cut edges in the rear region of the article that are allowed to move apart in order to provide enhanced fit to the body and better conformability to the wearers anatomy, particularly in the rear region, where the article typically extends through the posterior perineal area towards the groove between the buttocks. Such tridimensional disposable absorbent articles are preferably also provided with a tridimensional structure capable of matching the non-linear grooves and the non-planar surfaces of the female body. The tridimensional disposable absorbent articles are described below by reference to a sanitary napkin or catamenial.

The term "sanitary napkin", as used herein, refers to an article which is worn by females externally of the body and adjacent to the pudendal region and which is intended to absorb and contain the various body fluids which are discharged from the body (e.g., vaginal discharges, menses, and/or urine) and which is intended to be discarded after a single use. It should be understood, however that the present invention is also applicable to other feminine hygiene or catamenial pads such as pantiliners, or other absorbent articles such as incontinence pads, and the like.

The term "use", as used herein, refers to the period of time that starts when the absorbent article is actually put in contact with the anatomy of the user.

The terms "joined" or "affixed", as used herein, encompasses configurations whereby a first member is directly connected to a second member and configurations whereby a first member is indirectly connected to a second member by connecting the first member to intermediate members which in turn are connected to the second member.

As used herein, the term "pudendal" refers to the externally visible female genitalia and is limited to the labia majora, the labia minora, the clitoris, and the vestibule.

According to the present invention, a disposable absorbent article is disclosed, which has prior to use a three dimensional shape capable of conforming to the various complex body shapes of the female anatomy comprising non-linear grooves and non-planar surfaces, in order to provide increased body fit and comfort, better fluid management, and reduced leakage. The tridimensional shape is preferably inherent to the disposable absorbent article of the present invention, that is, inherently owned by the structure, such that it cannot be achieved starting from an initially flat product, e.g. by folding or pleating it. The disposable absorbent article of the present invention is intended for direct application to the users body, before an undergarment is worn, and further comprises a panty fastening system for attaching the article to said undergarment, wherein this panty fastening system comprises an adhesive means that can be activated, in order to actually provide adhesion between the article and the undergarment, only after the undergarment has been worn.

By the term "to wear" related to an undergarment, e.g. in the expressions "before an undergarment is worn", or "after an undergarment is worn", as used herein, it is meant that the undergarment is completely pulled up on the users body and is applied or positioned in the vicinity of the general urethral/genital area of the user.

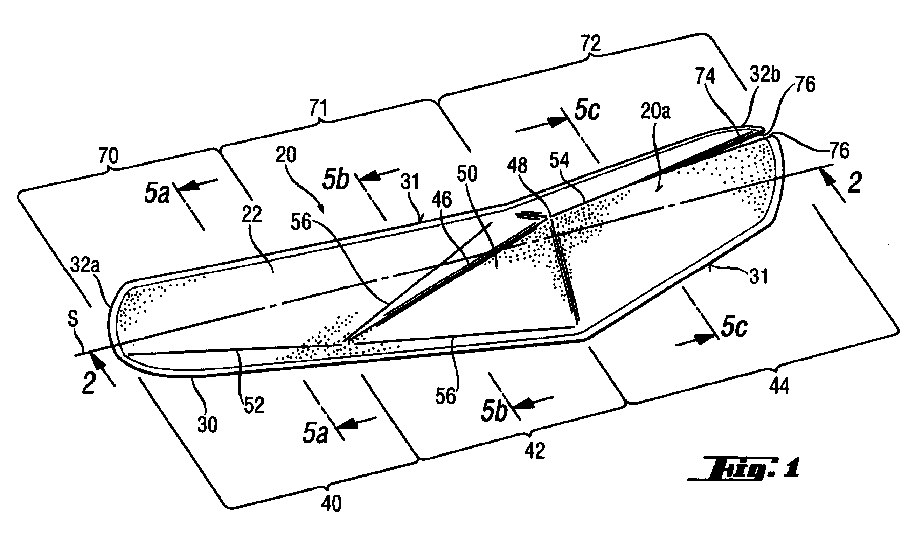

FIG. 1 is a perspective view of a sanitary napkin 20 of the present invention with its preferred tridimensional structure before use, with most of the portion of the sanitary napkin 20 that faces or contacts the wearer, oriented towards the viewer. By saying "before use", it is meant that the preferred sanitary napkin 20 of the present invention is provided with a tridimensional structure before it is actually worn, i.e., applied to the user's body. The sanitary napkin can nevertheless be packaged in a folded flat configuration, being subsequently unfolded to get the tridimensional shape just before wearing it. As better shown in FIG. 2, the sanitary napkin 20 comprises a liquid pervious topsheet 22, a liquid impervious backsheet 23 joined with the topsheet 22, and an absorbent core 24 positioned between the topsheet 22 and the backsheet 23.

The sanitary napkin 20 has two surfaces, a body facing or contacting surface 20a and a garment facing or contacting surface 20b. The body contacting surface 20a is intended to be worn adjacent to the body of the wearer while the garment surface 20b is on the opposite side and is intended to be directed towards the undergarment when the sanitary napkin 20 is worn, e.g. placed against it. Corresponding body facing and garment facing surfaces can also be identified in each single layer that constitutes the sanitary napkin 20, e.g., in the absorbent core 24. The sanitary napkin 20 has a longitudinal symmetry plane S. The term "longitudinal", as used herein, refers to a line, axis or direction in the sanitary napkin 20 that is generally aligned with (e.g., approximately parallel to) a vertical plane which bisects a standing wearer into left and right body halves when the sanitary napkin 20 is worn. The symmetry plane S of the sanitary napkin 20 substantially corresponds to this vertical plane that bisects the standing wearer. While it is preferred that the sanitary napkin 20 is exactly divided by the longitudinal symmetry plane S into two symmetrically equal halves, it is not excluded that the two halves be not specular. The term "transverse", as used herein, refers to a direction that is generally perpendicular to the longitudinal symmetry plane. The term "longitudinally oriented" refers to a direction, as seen in plan view, comprised within ±45 degrees, of the longitudinal symmetry plane S; the term "transversely oriented" similarly refers to any other direction, as seen in plan view.

The terms "front" and "rear', as used herein, refer to portions or edges in the sanitary napkin 20 that are oriented towards the front and rear part of the wearer's body, respectively, when the sanitary napkin 20 is being worn.

The sanitary napkin 20 has a periphery 30, that is defined by the outer edges of the sanitary napkin 20. The longitudinal edges 31 of the sanitary napkin 20 are aligned with the longitudinal symmetry plane S, and the ends edges of the sanitary napkin 20 comprise a front end edge 32a and a rear end edge 32b. The absorbent core 24 of the sanitary napkin has a front portion 40, a central portion 42 and a rear portion 44, each one preferably corresponding to approximately one third of the total length of the absorbent core 24. A front region 70, a central region 71, and a rear region 72 are identified in the sanitary napkin 20, respectively corresponding to the front portion 40, the central portion 42, and the rear portion 44 of the absorbent core 24.

In the preferred embodiment of the present invention the tridimensional sanitary napkin 20 is provided prior to use with a tridimensional structure that is intended to match the complex body shapes of the female anatomy. The tridimensional structure has preferably a structural tridimensionality, by "structural tridimensionality" being meant that the structure cannot be completely flattened onto a flat surface while keeping its integrity, that is, without being in any case e.g. torn, crushed or squeezed. In other words, the tridimensional structure cannot be achieved by simply folding or pleating an initially flat article, but is inherently owned by the absorbent article according to the present invention. The tridimensional sanitary napkin 20 of the present invention has preferably a substantially constant thickness, that is more preferably less than 5 mm; the sanitary napkin can be therefore considered of the thin type.

While the topsheet, the backsheet, and the absorbent core may be assembled in a variety of well known configurations (including so called "tube" products or side flap products), FIG. 1 shows a preferred embodiment of the sanitary napkin 20 in which the topsheet 22 and the backsheet 23 have length and width dimensions generally larger than those of the absorbent core 24. The topsheet 22 and the backsheet 23 extend beyond the edges of the absorbent core 24 to thereby form the periphery 30 of the sanitary napkin 20.

The topsheet 22 is compliant, soft feeling, and non-irritating to the wearer's skin. Further, the topsheet 22 is liquid pervious, permitting liquid (e.g. menses and/or urine) to readily penetrate through its thickness. A suitable topsheet 22 may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibres (e.g., wood or cotton fibers), synthetic fibres (e.g., polymeric fibres such as polyester, polypropylene, or polyethylene fibres); or from a combination of natural and synthetic fibres.

A preferred topsheet comprises an apertured formed film. Apertured formed films are preferred for the topsheet because they are perilous to body fluids and yet non-absorbent and have a reduced tendency to allow liquids to pass back through and rewet the wearer's skin. Thus, the surface of the formed film which is in contact with the body remains dry, thereby reducing body soiling and creating a more comfortable feel for the wearer.

Suitable formed films are described in U.S. Pat. No. 3,929,135, issued to Thompson on December 30, 1975; U.S. Pat. No. 4,324,246, issued to Mullane, et al. on April 13, 1982; U.S. Pat. No. 4,342,314, issued to Radel, et al. on August 3, 1982; U.S. Pat. No. 4,463,045, issued to Ahr, et al. on July 31, 1984; and U.S. Pat. No. 5,006,394, issued to Baird on April 9, 1991. A preferred topsheet for the absorbent article of the present invention is a formed film described in one or more of the above patents and marketed on sanitary napkins by The Procter & Gamble Company of Cincinnati, Ohio as "DRI-WEAVE".

In a preferred embodiment of the present invention, the body or exposed surface of the formed film topsheet is hydrophilic so as to help liquid transfer through the topsheet faster than if the body surface were not hydrophilic so as to diminish the likelihood that menstrual fluid will flow off the topsheet rather than flowing into and being absorbed by the absorbent core. In a preferred embodiment, surfactant is incorporated into the polymeric materials of the formed film topsheet such as is described in U.S. Patent Application Serial No. 07/794,745, Aziz et al., filed on November 19, 1991. Alternatively, the body surface of the topsheet can be made hydrophilic by treating it with a surfactant such as is described in U.S. 4,950,254.

The absorbent core 24 may be any absorbent means that is capable of absorbing or retaining liquids (e.g., menses and/or urine). The absorbent core 24 may be manufactured in a wide variety of sizes and shapes (e.g., rectangular, oval, hourglass, asymmetric, etc.) and from a wide variety of liquid-absorbent materials commonly used in sanitary napkins and other absorbent articles such as comminuted wood pulp that is generally referred to as airfelt. Examples of other suitable absorbent materials include creped cellulose wadding, modified cross-linked cellulose fibres (such as those described in U.S. Patent No. 5,217,445 issued to Young, et al. on June 8, 1993), capillary channel fibres (that is, fibres having intra-fibre capillary channels such as those described in U.S. Patent No. 5,200,248 issued to Thompson, et al. on April 6, 1993), absorbent foams (such as those described in U.S. Patent No. 5,260,345, issued to DesMarais, et al. on November 9, 1993 and U.S. Patent No. 5,268,244 issued to DesMarais, et al. on December 7, 1993), thermally bonded airlaid materials (such as those material described in U.S. Patent Application Serial No. 08/141,156, entitled "Catamenial Absorbent Structures Having Thermally Bonded Layers For Improved Handling of Menstrual Fluids and Their Use In Catamenial Pads Having Improved Fit and Comfort" filed in the name of Richards, et al. on October 21, 1993), absorbent sponges, synthetic staple fibres, polymeric fibres, hydrogel-forming polymer gelling agents, peat moss, tissue including tissue wraps and tissue laminates, or any equivalent materials or combinations of materials. Suitable absorbent cores comprising foams are described in European Applications 0 598 833, 0 598 823 and 0 598 834. Suitable absorbent cores comprising tissue laminates with particles of hydrogel-forming polymer gelling agents comprised therebetween are described in International Patent Applications WO 94/01069 and WO 95/17868.

The configuration and construction of the absorbent core may also be varied (e.g., the absorbent core may have varying caliper zones, e.g., profiled so as to be thicker in the centre), hydrophilic gradients, superabsorbent gradients, or lower density and lower average basis weight acquisition zones; or may comprise one or more layers or structures. The total absorbent capacity of the absorbent core should, however, be compatible with the design leading and the intended use of the sanitary napkin. Further, the size and absorbent capacity of the absorbent core may be varied to accommodate different uses such as incontinence pads, pantiliners, regular sanitary napkins, or overnight sanitary napkins. Preferably the absorbent articles of the present invention are sanitary napkins which are uniform in thickness.

The backsheet 23 and the topsheet 22 are positioned adjacent the garment facing surface 20b and the body facing surface 20a, respectively, of the absorbent core 24 and are preferably joined thereto and to each other by attachment means (not shown) such as those well known in the art. For example, the backsheet 23 and/or the topsheet 22 may be secured to the absorbent core 24 or to each other by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota under the designation HL-1258 or H-2031. The attachment means will preferably comprise an open pattern network of filaments of adhesive as is disclosed in U.S. Patent 4,573,986 entitled "Disposable Waste-Containment Garment", which issued to Minetola, et al. on March 4, 1986. An exemplary attachment means of an open pattern network of filaments comprises several lines of adhesive filaments swirled into a spiral pattern such as illustrated by the apparatus and method shown in U.S. Patent 3,911,173 issued to Sprague, Jr. on October 7, 1975; U.S. Patent 4,785,996 issued to Zieker, et al. on November 22, 1978; and. U.S. Patent 4,842,666 issued to Werenicz on June 27, 1989. Alternatively, the attachment means may comprise heat bonds, pressure bonds ultrasonic bonds, dynamic mechanical bonds, or any other suitable attachment means or combinations of these attachment means as are known in the art.

The backsheet 23 is impervious to liquids (e.g., menses and/or urine) and is preferably manufactured from a thin plastic film, although other flexible liquid impervious materials can also be used. In use, the backsheet 23 is interposed between the absorbent core 24 and the user's undergarments. The function of the backsheet 23 is to prevent exudates which may be expelled from or which inadvertently bypass the absorbent core 24 from contacting and soiling the user's undergarments. The backsheet 23 can thus comprise a woven or nonwoven material, polymeric films such as thermoplastic films of polyethylene or polypropylene, or composite materials such as a film-coated nonwoven material. Preferably, the backsheet is a polyethylene film having a thickness of from about 0.012 mm to about 0.015 mm. Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet 23 is preferably embossed and/or matte finished to provide a more clothlike appearance. Further, the backsheet 23 may permit vapours to escape from the absorbent core 24 (i.e., it can be breathable) while still preventing exudates from passing through the backsheet 23.

As illustrated in FIGS. 1 and 2, the tridimensional sanitary napkin 20 in its preferred embodiment has before use a tridimensional structure with a longitudinal oriented ridge 50 in the central and rear portions 42, 44 of the absorbent core 24, such that the line of intersection 46 of the longitudinal symmetry plane S with the body facing surface 20a has a slope decreasing rearwardly, i.e. towards the rear end edge 32b, in the central portion 42 and in the rear portion 44 of the absorbent core 24. This can be seen more clearly in FIG. 2, where the longitudinal sectional view of the sanitary napkin 20 shows the line of intersection 46 with its decreasing slope in the central and rear portions 42, 44.

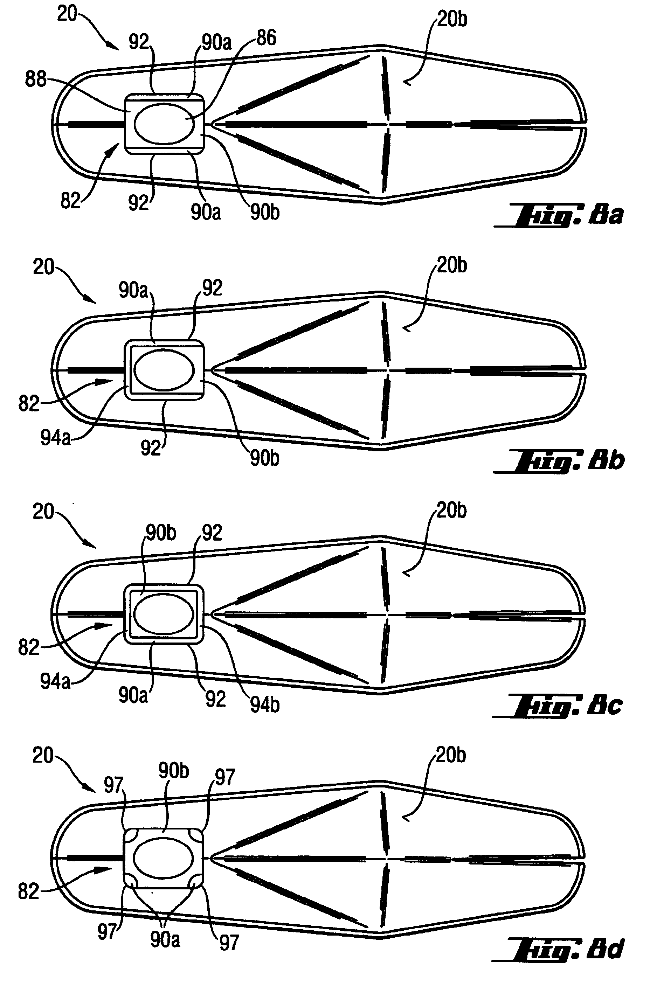

According to a preferred embodiment of the present invention the sanitary napkin 20 further comprises a cut or slit 74 in its rear region 72, extending from the rear end edge 32b and, in the preferred embodiment illustrated in FIGS. 1 to 3, being aligned with the longitudinal symmetry plane S. The cut or slit 74 affects the whole structure of the sanitary napkin 20, i.e., in the illustrated embodiment, the topsheet 22, the absorbent core 24 and the backsheet 23, so defining corresponding cut edges 76. It is preferred that the structure of the sanitary napkin 20 is sealed along the cut edges 76, e.g. by joining together the topsheet 22 and the backsheet 23 by means of adhesive, or heat, or any other known means, so that portions of the cut absorbent core 24 are not exposed along the cut edges 76. The cut or slit 74 allows the cut edges 76 of the sanitary napkin 20 to move apart form each other along said cut or slit 74, so that the longitudinally oriented, preferably inverted V shaped structure of the ridge 50 can bend at any point along the cut or slit 74 around an axis that is perpendicular to the symmetry plane S, as better shown in FIGS. 7a and 7b, where a slightly different embodiment of the present invention is illustrated, in order to better fit the body anatomy typically in the region of the gluteal groove, where the rear region 72 of the tridimensional sanitary napkin 20 preferably extends in order to provide a better rearward protection.

More in detail, FIGS. 7a and 7b illustrate the same sanitary napkin 20 in two different embodiments of the rear region 72, corresponding to two different wearing situations, where the longitudinally oriented, inverted V shaped structure of the ridge 50 bends at two different points around an axis perpendicular to the symmetry plane S. This can be due to the adaptation of the article to different anatomies, i.e. to wearers having different body shape in the region of the gluteal groove, e.g., FIG. 7b could show the configuration of a sanitary napkin 20 while worn by a smaller wearer. Or, alternatively, this can be caused by e.g. different forces acting on the sanitary napkin during the use.

The cut or slit 74 therefore provides the structure of the inverted V shaped ridge 50, which would be per se less capable of bending around an axis perpendicular to the symmetry plane S without creasing and/or bending away form the body, and/or exerting a force on the remaining portions of the sanitary napkin 20 extending forward of said rear region 72, with the capability of adapting to the various body shapes, particularly in the region of the gluteal groove, therefore adjusting to different groove lengths and radii of curvature, and also to changes that typically occur with time in the same wearer, e.g. through movements.

In the preferred embodiment of the present invention illustrated in FIGS. 1 to 7 the line of intersection 46 has a preferred profile with a rearwardly decreasing slope as seen in cross-sectional view, as will be explained more in detail below.

The decreasing slope of said line of intersection 46 can be expressed mathematically if said line of intersection 46 is considered in a Cartesian x-y system lying in the symmetry plane S, wherein the x-axis is defined by the two points of intersection of the longitudinal symmetry plane S with the front end edge 32a and the rear end edge 32b of the sanitary napkin 20, substantially corresponding to the points indicated by numerals 32a and 32b in the cross-section view of the sanitary napkin 20 illustrated in FIG. 2, and wherein the body facing surface 20a faces towards positive y values.

With respect to this system of axes one can form the first derivative of the line of intersection 46. According to the present invention, the first derivative of this line 46 in the longitudinal direction has at least one value that is larger in the central portion 42 of the absorbent core 24 than at least one value in the rear portion 44 of the absorbent core 24. This includes the preferred case, illustrated in FIGS. 1 and 2, where the intersection line 46 is always inclined upward towards the rear end edge 32b with two different slopes in the central portion 42 and in the rear portion 44, and also alternative embodiments in which, e.g., the line of intersection 46 is inclined upward in the central portion 42 and downward in the rear portion 44.

The consecutive values of the first derivative of the line of intersection 46 can decrease continuously towards the rear end edge 32b, implying that the line of intersection 46 has a curved profile with a continuously decreasing slope, or, alternatively, the first derivative can assume different discrete values along the length of the intersection line 46. For example, it can be constant in either the central portion 42, or in the rear portion 44, or in both, the latter being the case of the embodiment illustrated in FIGS. 1 and 2, where the intersection line 46 is formed by two substantially rectilinear portions having constant slopes, with a slope change at a point 48 of the line of intersection 46 positioned where the central portion 42 of the absorbent core 24 merges the rear portion 44.

In the preferred embodiment of the present invention illustrated in FIGS. 1 to 3 the cut or slit 74 extends from the rear end edge 32b substantially along the symmetry plane S. Therefore no actual line of intersection 46 can be identified where the cut edges 76 are moved apart form each other, but only where no cut or slit exists, or also where the cut edges 76 are kept close to each other and no displacement of them occurs. This is typically the case of the tridimensional sanitary napkin 20 of the present invention since the bending axis perpendicular to the symmetry plane S can move along the cut or slit 74 according to the different anatomies and/or to the changes experienced during the wearing time.

In the preferred embodiment of the present invention herein described the sanitary napkin 20 features a line of intersection 46 with the preferred profile, that is kept with any possible bent configuration of the rear region 72 since the cut or slit 74 does not extend up to the point 48 where the slope change in the line of intersection 46 occurs. In other words, even if the cut edges 76 of the rear region 72 of the sanitary napkin 20 are caused to move apart along the entire length of the cut or slit 74, the longitudinal oriented ridge 50 still comprises a line of intersection 46 with the preferred slope decreasing rearwardly. In this preferred embodiment the sanitary napkin 20 therefore keeps its preferred structural tridimensionality wherever the bending axis perpendicular to the symmetry plane S is located along the cut or slit 74 in the rear region 72.

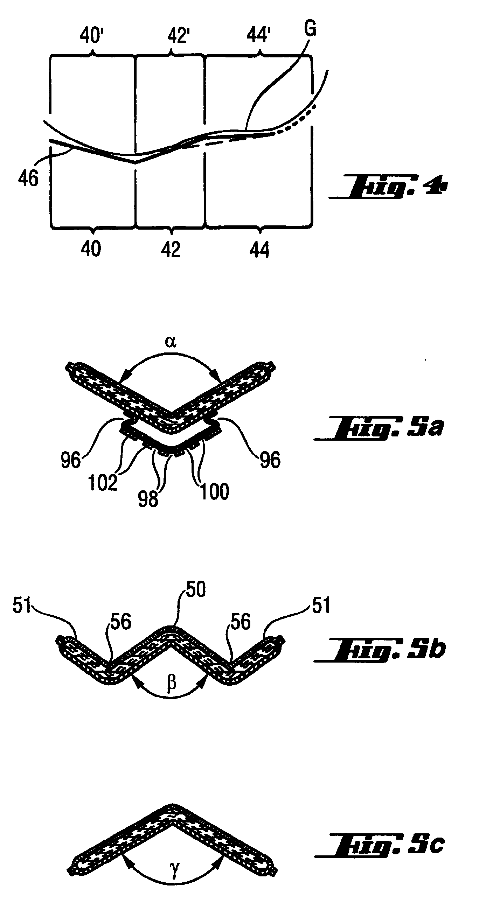

A line of intersection 46 with the above described profile in combination with the cut or slit 74 in the rear region 72 provides the preferred sanitary napkin 20 of the present invention with a longitudinally oriented ridge 50 in the central and rear portions 42, 44 of the absorbent core 24. The ridge 50 has a longitudinal non linear profile that is intended to match in use the central non linear groove of the female anatomy extending from the labia majora to the perineum and into the gluteal groove, and having approximately the shape schematically indicated in the corresponding central and rear portions 42', 44' of the curve G, also featuring a corresponding front portion 40', illustrated in FIG. 4, where the matching profile of a line of intersection 46 in a sanitary napkin illustrated in FIGS. 1 to 3 is also shown. The cut or slit 74 in the rear region 72 also provides the longitudinally oriented ridge 50 with the capability of bending around an axis perpendicular to the symmetry plane S and positioned at any location along the cut or slit 74 itself, in order to better fit the different body anatomies and the varying in use conditions in the region of the gluteal groove.

The dotted line following the rearward portion of the profile of the line of intersection 46 in FIG. 4 corresponds to the portion of the rear cut or slit 74 where the cut edges 76 are actually displaced from each other during the use of the sanitary napkin 20 of the present invention. Such dotted line does not correspond to an intersection line since, as already explained, there can be no intersection between the symmetry plane S and the body facing surface 20a where the cut edges are caused to move apart. It rather indicates the actual profile described by one of the cut edges 76 in the rear region 72 of the sanitary napkin 20 as seen in cross-sectional view, where the upwardly convex, inverted V shaped rear region of the sanitary napkin 20 is allowed to bend along an axis perpendicular to the symmetry plane S in order to better fit the body anatomy in the region of the gluteal groove, corresponding to the rearmost part of the rear portion 44' of the curve G. The profile shown in FIG. 4 can be considered as substantially corresponding to a sanitary napkin having the configuration illustrated in FIG. 7a, with the bending axis perpendicular to the symmetry plane S located closer to the rear end edge 32b.

The profile of the longitudinally oriented ridge 50 as defined by the line of intersection 46 with its slope decreasing rearwardly, and in combination with the cut or slit 74 in the rear region 72 can provide the sanitary napkin 20 with an improved fit to the wearers body. In the preferred embodiment illustrated in FIG. 1, when going from front to rear, the forward portion of the ridge 50, with a substantially constant slope, is intended to fit the groove between the labia majora. The subsequent portion of the ridge 50 that bridges the central and the rear portions 42, 44 of the absorbent core 24, with its change in slope, has a profile that is capable of matching in use the downwardly concave portion of the central non linear groove of the female anatomy in the region going from the rearward part of the labia majora to the perineum, so as to achieve a continuous contact with the body. This provides for a better comfort and for a more effective interception of the fluids as they are released from the body. The rearward portion of the longitudinally oriented ridge 50, still belonging to the rear portion 44 of the absorbent core 24 and having a constant slope in the embodiment of FIG. 1, is intended to extend between the buttocks in the gluteal groove. Owing to its slighter slope, as compared to the forward portion of the ridge, it is capable of contacting the body without causing any stress between the anatomy and this portion of the sanitary napkin, which could in turn cause discomfort, and/or prevent the desired substantially continuous contact between the ridge 50 and the wearer's anatomy along the entire length of the non linear groove extending from the labia majora up to the gluteal groove. Finally, the rearmost portion of the ridge 50, characterized by the presence of the cut or slit 74, can bend, typically upwardly, around an axis perpendicular to the symmetry plane S, in order to better fit the body shape in the area of the gluteal groove, where the rear region 72 of the sanitary napkin 20 preferably extends to provide an increased protection, e.g. against rear leakage that can be experienced during motion or during the sleep, when the wearer lies on her back. This provides for an improved fit of the sanitary napkin 20 in the area of the gluteal groove, also avoiding any possible negative interaction with the undergarment, that could otherwise exert a force on the upwardly convex rear region of the sanitary napkin 20 extending rearwardly, therefore causing said upwardly convex region to crease or move away from the body.

A ridge 50 with a preferred profile having a slope decreasing rearwardly can get further into the non linear groove of the female anatomy, as schematically indicated in FIG. 4. The ridge 50 with the profile indicated by the line 46 is in fact capable of following the profile of the groove, indicated by the curve G, by extending past a line, indicated with the dashed line in FIG. 4, that connects two points along the central groove of the body surface where the sanitary napkin has contact with the anatomy, e.g. the two points where the sanitary napkin contacts the body in correspondence of the forward and rearward portions of the ridge. The rear end of the dashed line actually goes up to the rearmost portion of the ridge 50 where the cut edges 76 are displaced form each other; it therefore corresponds to the beginning of the dotted line, as explained hereinbefore, following the profile of the line of intersection 46. A ridge shaped with a linear profile as those known in the art cannot extend past this line, since such a ridge substantially corresponds to this line, and hence cannot provide a continuous contact with the body along the entire length of the ridge.

Of course the situation described so far of a preferred tridimensional sanitary napkin 20 and its interaction with the wearers anatomy represents only a particular preferred embodiment of the present invention, that is intended to indicate the general capability of the ridge 50 with the preferred longitudinal non linear profile, preferably in combination with the rear cut or slit 74, to match in use along its entire length the central non linear groove of the female anatomy, therefore providing for a better contact with the body and an increased comfort.

In the preferred embodiment of the present invention illustrated in FIGS. 1 and 2 the tridimensional sanitary napkin 20 preferably has a low constant thickness that is less than 5 mm, wherein the tridimensional structure is provided without the use of humps or of regions of different thickness, and it is an inherent feature of the sanitary napkin 20, rather than an added feature, achieved e.g. by bending, folding or joining together an initially planar structure.

As shown in the embodiment of the present invention illustrated in FIGS. 1 and 2, the front portion 40 of the absorbent core 24 is preferably upwardly concave, in order to better conform to the wearer's anatomy in the region of the mons pubis.

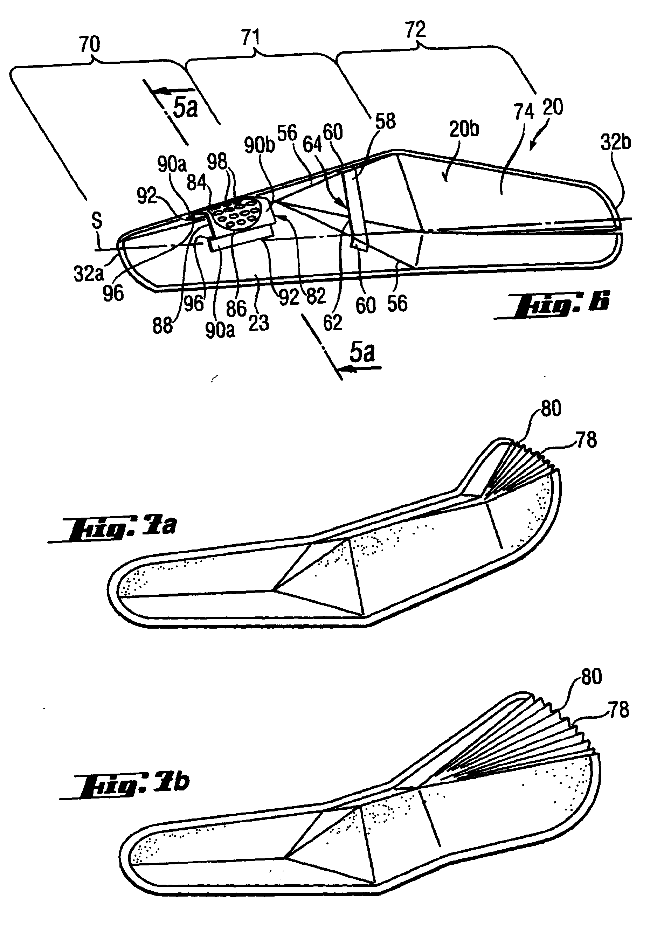

The sanitary napkin 20 illustrated in FIGS. 1 and 2 shows a particularly preferred configuration for the front, central and rear portions 40, 42, and 44 of the absorbent core 24. As viewed in transverse section the front, central and rear portions of the absorbent core 24 have respectively a V shape, a W shape, and an inverted V shape, as better shown in FIGS. 5a, 5b, and 5c, where transverse sections of the sanitary napkin 20 taken on lines 5a-5a, 5b-5b, and 5c-5c respectively of FIG. 1 are illustrated.

These different shapes provide the sanitary napkin 20 with the further capability of conforming to the wearer's anatomy in a direction substantially perpendicular to the already defined symmetry plane S. The V shape of the front portion 40 and the inverted V shape of the rear portion 44 merge together gradually in the central portion 42, where the resulting W shape is predisposed to fit the area of the labia majora and of the perineum. In use, the longitudinally oriented ridge 50 is intended to fit the longitudinal central groove as above described, while the side portions 51 bent upwardly can match the groin creases, i.e. the two grooves that are formed between the body and the legs, typically in the area where the panty elastics contact the body.

In the preferred embodiment of the present invention illustrated in FIGS. 1 and 2 the sanitary napkin 20 with its three dimensional shape prior to use is provided with an increased capability of conforming to the wearer's anatomy than that simply given by the known differentiated transverse shaping of the different portions of the absorbent core 24.

The tridimensional structure of the sanitary napkin 20 already present in the article prior to use is such that the width of the angle γ of the inverted V shaped portion increases towards the rear end edge 32b of the sanitary napkin 20 starting from a minimum preferred value at a position corresponding to the merging of the rear portion 44 with the central portion 42 of the absorbent core 24, where it substantially corresponds to the angle β of the central inverted V part of the W shaped central portion 42, which is in turn substantially constant along the entire length of this portion 42. Therefore the rearward portion of the ridge 50, typically positioned in use between the buttocks, can more easily widen its inverted V shape during the wearing of the product without being restrained, so providing the sanitary napkin with a better conformability to the anatomical configuration of the wearer. Of course the further feature of the cut or slit 74 in the rear region 72 of the sanitary napkin 20, not shown in FIG. 5c, which is a section taken at a location where the cut or slit 72 is not present, allows the cut edges 76 of the rearmost portion of the ridge 50 to move apart form each other to even better fit the region of the gluteal groove, so adapting to different lengths and radii of curvature of said groove.

A feature similar to that described for the rear portion 44 is preferably provided in the V shaped front portion 40 of the absorbent core 24, where the angle α of the V increases its width towards the front end edge 32a of the sanitary napkin 20 from a minimum preferred value at a point corresponding to the merging of the front portion 40 with the central portion 42. This will allow the portion of the sanitary napkin 20 which is closer to the front end edge 32a to more easily flatten in transverse direction during wearing in order to accommodate the relatively flat front part of the mons pubis, while still providing an overall concave shape to effectively follow the surface of the mons pubis.

The angles of the V shaped front portion 40 and/or of the inverted V shaped rear portion 44 of the absorbent core 24, and consequently of the entire sanitary napkin 20, can therefore increase towards respective end edges 32a and/or 32b up to values around 180°, in order to better accommodate the anatomy of the wearer without inducing any substantial stress in the structure, thus providing for a better fit and comfort.

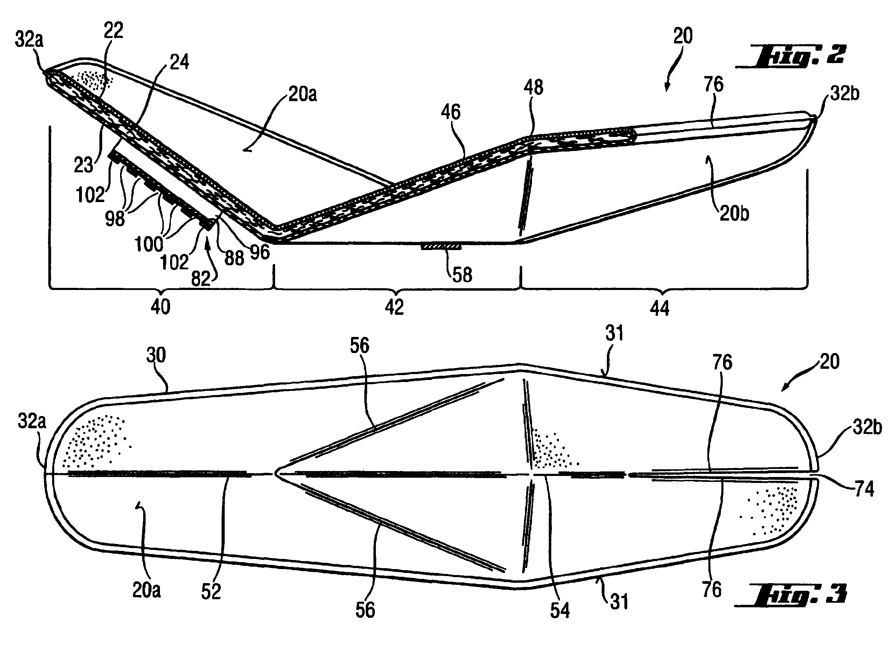

The preferred feature of the angles increasing towards respective end edges in the V shaped and inverted V shaped portions is achieved by giving the front portion 40 and/or the rear portion 44 of the absorbent core 24 a cup shaped structure with any means known to the man skilled in the art. For example, in the sanitary napkin 20 of the present invention illustrated in FIGS. 1 and 2 this is achieved by cutting away a narrow V shaped portion of material centered along the longitudinal centreline of initially flat front portion 40 and rear portion 44 of the absorbent core 24, and of the topsheet 22 and the backsheet 23 as well, and having substantially the same length of the front portion 40 and of the rear portion 44, and then joining together the cut edges with known means, e.g. by thermobonding, along the junction lines identified as 52 and 54 in FIG. 3. Of course the junction line 54 does not extend up to the rear end edge 32b when the preferred cut or slit 74 has to be formed. The final tridimensional structure illustrated in FIGS. 1 and 2 is then achieved by suitably bending the non planar sanitary napkin 20, e.g. along lines of preferential bending, formed in the absorbent core 24 by means of e.g. embossments or partial cuts, such as the embossments 56 in FIG. 3, as can be readily determined by the man skilled in the art. In this preferred embodiment the cup shaped structure of the central and rear portions 42, 44 of the absorbent core 24, and therefore of the corresponding rear region 72 of the sanitary napkin 20, is intrinsically stable, i.e., has the already defined structural tridimensionality that is not hindered by the rear cut or slit 74, since it does not run the whole length of the rear portion 44 of the absorbent core 24 up to the peak 48.

The presence of the above described preferred feature in the sanitary napkin of the present invention illustrated in FIGS. 1 and 2 can be readily ascertained when folding transversely the sanitary napkin 20 in order to superimpose the front portion 40 or the rear portion 44 of the absorbent core over the central portion 42 along a fold line that approximately in the unfolded sanitary napkin corresponds to a line separating respectively the front portion 40 or the rear portion 44 from the central portion 42: in both cases the folding line will show an angle rather than being rectilinear.

The combination of the tridimensional structure of the sanitary napkin 20 of the present invention, comprising the longitudinally oriented ridge 50 with the preferred profile of the line of intersection 46, preferably with the rear cut or slit 74 in the rear region 72, provides the sanitary napkin 20 with an increased capability to fit to the non-planar surfaces and the non-linear grooves of the female anatomy, along the entire length of the sanitary napkin. The improved fit achieved by a tridimensional sanitary napkin, particularly a tridimensional sanitary napkin in the preferred embodiment described so far, is capable of providing a proper and stable positioning of the sanitary napkin during the use. There is therefore no risk that the sanitary napkin is mispositioned with respect to the anatomy, or is moved from its preferred location during the use. This provides that in use the liquid is properly received and acquired in an acquisition zone preferably located in the central portion 42 of the absorbent core 24, typically forward of the peak 48 of the ridge 50, while the rear region of the sanitary napkin preferably characterized by the slit or cut is positioned rearwardly of this acquisition zone. The cut or slit 74, in turn, being oriented substantially longitudinally, does not create any obstacle to the diffusion of the liquid within the absorbent core 24, which itself occurs in a preferred path oriented in longitudinal direction.

The rear cut or slit 74 can therefore be left completely open, with no need of an additional material joining the cut edges 76.

In an alternative preferred embodiment of the present invention, however, illustrated in FIGS. 7a and 7b, the sanitary napkin 20 can comprise a material 78 that joins the cut edges 76 of the rear cut or slit 74, and that allows the cut edges 76 to move apart. Said material 78 is preferably liquid impervious, therefore providing the article with an added protection in the rear region 72 with the cut or slit 74. Said material 78 shall be provided by any known means with the capability of allowing the cut edges 76 to move apart, e.g. it can be extensible, elastic, or pleated, as illustrated in FIGS. 7a and 7b, where a liquid impervious plastic film 78 provided with pleats 80 and joining the cut edges 76 along their entire length is shown. The material 78 can be a separate element added to the structure of the sanitary napkin 20, e.g. joined to the backsheet 24, or can be integral with the structure, being e.g. a portion of the backsheet 24 made extensible by known means.

In the preferred embodiments of the present invention shown in FIGS. 1 to 7 the sanitary napkin 20 always comprises a single rear slit or cut 74 in its rear region 72, extending from the rear end edge 32b substantially along the longitudinal symmetry plane S. In alternative embodiments of the present invention more than one cut or slit can be provided in the rear region of a tridimensional absorbent article. In further alternative embodiments the at least one cut or slit does not necessarily run along the longitudinal symmetry plane S of the tridimensional absorbent article, provided that the at least one cut or slit extends from the rear end edge in a direction towards a point located on the longitudinal symmetry plane S.

In an alternative embodiment of the present invention a tridimensional shape similar to that illustrated in FIGS. 1 to 5c can also be achieved by comprising in a disposable absorbent article a resilient insert having the desired shape, e.g. between the backsheet and the absorbent core. The insert can be comprised for example only in the central and rear portions of the absorbent article, where the ridge with the desired profile is to be provided, or can extend along the entire length of the absorbent article, in order to provide its whole shape. The resilient insert can be made of any known suitable material, e.g. absorbent or non absorbent material, and can be produced e.g. by thermoforming to get the desired tridimensional shape, preferably with a constant thickness. The insert can completely provide the tridimensional structure, or can alternatively contribute to create and to maintain said structure in an already shaped absorbent article. The insert can also comprise the cut or slit.

The sanitary napkin 20 of the present invention having the preferred embodiment illustrated in FIGS. 1 to 5c and described hereinbefore is intended to be applied by the user directly to the body, and preferably also comprises means 58 for holding and applying it located on the garment facing surface 20b and being oriented transversely, as those described in European patent application EP 97110734.7. As illustrated in FIG. 6, a perspective view of the sanitary napkin 20 of FIG. 1 is shown, as seen from the side that lies remote from the wearer in use, i.e., with the garment facing surface 20b towards the viewer. The means 58 for holding and applying the sanitary napkin 20 are also referred to hereinbelow as a handling aid.

Of course the means 58 for holding and applying the sanitary napkin 20 of the present invention are also intended for use by a person taking care of a user, e.g. a nurse, who handles the sanitary napkin 20 and applies it to the user's body.

In the preferred embodiment of FIG. 6 the means 58 for holding and applying the sanitary napkin 20 comprises an elongated strip of elastic film material 58 oriented perpendicularly to the longitudinal symmetry plane S and located on the garment facing surface 20b of the sanitary napkin 20, in correspondence of the central portion 42 of the absorbent core 24, at a position approximately longitudinally intermediate between the front end edge 32a and the rear end edge 32b of the sanitary napkin 20. The strip 58 is affixed to the backsheet 23 at its two spaced apart ends 60 disposed on opposite sides of the symmetry plane S, with an intermediate portion 62 being not joined to said garment facing surface 20b and defining a space 64, intended for the insertion of at least one user's finger for holding and applying the sanitary napkin 20. In the embodiment illustrated in FIG. 6, where the sanitary napkin 20 has the preferred tridimensional shape before use, the space 64 is actually comprised between the intermediate portion 62 of the strip 58 and the garment facing surface 20b of the central portion of the sanitary napkin, which is concave on its garment facing surface 20b, since it corresponds to the ridge 50 on the body facing surface 20a. Typically the spaced apart ends 60 of the strip 58 are fixed with known means, e.g., with an adhesive, or by thermobonding, to the garment facing surface 20b of the backsheet 23 at intermediate locations between each bend line corresponding to the embossments 56, and the respective longitudinal edge 31.

The user can put the sanitary napkin 20 on the palm of her hand with the garment facing surface 20b contacting the hand and with the front end edge 32a facing towards the wrist, at the same time inserting typically one of her fingers, e.g. the middle finger, in the space 64 between the intermediate portion 62 of the strip 58 and the backsheet 23. The user can therefore hold the sanitary napkin 20 in her open hand without exerting any force, also owing to the elasticity of the preferred material that constitutes the strip 58, with substantially the front portion of the sanitary napkin 20 lying on her palm. Application to the body can then be easily performed by the user with a single movement of her open hand, which is simple and self-explanatory as putting the empty hand on the body.

Moreover, the movements of the hand and of the fingers allow the user to completely control the manipulation of the sanitary napkin 20 during its application to the body, making use of the tactile sensitivity of the fingers to find the right position for the sanitary napkin 20. Particularly, in the preferred embodiment of the present invention, the finger inserted in the space 64 is substantially aligned with the ridge 50 on the body facing surface 20a of the sanitary napkin 20, and therefore can provide guidance to control the proper placement of the napkin 20 on the body anatomy, i.e. with the ridge 50 suitably registered with the longitudinal non-linear groove of the female anatomy extending from the labia majora to the gluteal groove. The forward portion of the ridge can be e.g. easily identified by the user with her finger inserted in the space 64, and used as a reference to direct the sanitary napkin into an optimal position on the body. The handling aid constituted by the strip 58 also allows an easy removal of the hand once the sanitary napkin 20 is in place, without disturbing or modifying the position of the napkin 20.

The tridimensional sanitary napkin 20 of the present invention comprises a panty fastening system, and therefore removal of the sanitary napkin 20 from the body and its possible subsequent temporary holding and reapplication to the body can be done by the user via the panty, by simply pulling the panty down and up again with the sanitary napkin adhered to it, for example to use the toilet, or to make a check of the article, or in any case in order to finally dispose of the product. However in principle, and based on the users preferences, the handling aid illustrated in FIG. 6 can also allow removal and, possibly, subsequent reapplication of the sanitary napkin 20 in the same situations mentioned above. The user can in fact for example pull down the panty while keeping the sanitary napkin 20 in contact with the body in order to cause its detachment from the panty, and then grab the sanitary napkin 20 while it is still being worn by positioning her hand substantially in the same way as for the application, with one of her fingers inserted in the space 64 between the not joined portion 62 of the strip 58 and the backsheet 23. The sanitary napkin 20 can therefore be taken off the body and held by the user; the handling aid may also be used to temporarily store the sanitary napkin, e.g. while using the toilet, on the user's hand, with no need for actually holding it with the fingers, or for exerting any force on it. Also in this case the panty fastening system of the present invention still provides the sanitary napkin 20 with a better capability to stay in place during the use, by preventing possible movements of the sanitary napkin 20 away from the desired position by means of the attachment to the panty, but without interfering with the body fit, as will be explained in detail hereinbelow.

The handling aid constituted by the strip 58 allows in any event the user to handle/manipulate the sanitary napkin 20 by contacting its garment facing surface 20b only, therefore protecting her hand from the possibly dirty body facing surface 20a.

In the preferred absorbent articles having a tridimensional shape before the use, such as the sanitary napkin 20 in the preferred embodiment described hereinbefore, the handling aid preferably also contributes to keep the tridimensional shape of the article during the use, e.g. in case of body movements that can disturb the proper fit of the product, or when in general there is a risk of collapse of the body fitting tridimensional shape. Otherwise the handling aid, e.g. constituted by the strip 58 illustrated in FIG. 6, stays aligned or folded or loose on the garment facing surface 20b of the product and does not disturb the product performance.

In alternative embodiments of the present invention the handling aid can be constituted by more than one strip of material, or by one or more strings, while the material can be also non elastic. The handling aid can be also constituted by a strip arranged as a loop and applied to the garment facing surface 20b of the article, or by a series of loops, intended to allow the insertion of at least one user's finger.

The handling aid can also be activated by the user, e.g. by being applied to the garment facing surface of the absorbent article just before use; alternatively, a handling aid e.g. constituted by a strip 58 can be detached e.g. at one of its ends from the garment facing surface of the absorbent article and then repositioned at a different place, in order to e.g. partially control or adapt a tridimensional shape already provided in the absorbent article, or to modify the space 64 available for the insertion of at least one user's finger. A handling aid preferably constituted by a strip 58 could therefore be resealably attached to the garment facing surface 20b of the absorbent article, at either one or both ends 60, e.g. by means of a resealable adhesive, or of a mechanical fastener of the hook and loop type, such as that marketed under the tradename VELCRO. A handling aid in form of a loop could be modified by the user in order to change the diameter of the loop, and hence the space available for the insertion of the finger(s).

In a further alternative embodiment of the present invention the disposable absorbent article can comprise a release cover releasably attached to the garment facing surface of the absorbent article, wherein the handling aid is located on said release cover. In use, after application of the absorbent article to the body by means of the handling aid, the release cover can be detached from the garment facing surface of the article, leaving e.g. an adhesive exposed, that can thus serve as a panty fastening means in a panty fastening system according to the present invention, as will be described hereinafter. Successive removal of the absorbent article would be performed by using the panty, with the now attached absorbent article, as an handling aid.

The handling aid does not necessarily extend across the entire width of the absorbent article, in order to define a suitable space for the insertion of at least one user's finger, which is capable of achieving a sufficiently firm fit with said at least one finger.

As illustrated in the preferred embodiment of FIG. 6, the handling aid does not extend in longitudinal direction over a major portion of the length of the disposable absorbent article; preferably, it extends over less than 10% of said length, being more preferably a narrow strip with a width, extending in said longitudinal direction, of about 1 cm.

According to the present invention, the tridimensional disposable absorbent article, namely the sanitary napkin 20 described so far, intended for direct application to the user's body before an undergarment, e.g. a panty, is worn, is provided with a panty fastening system 82 on the garment facing surface 20b of the backsheet 23, for attachment of the article to the undergarment during the use of the article itself, i.e., after the article has been applied to the body. The panty fastening system 82 according to the present invention comprises an adhesive means 84 which has a time delayed activation system, such that the activation of the adhesive properties of the adhesive is achieved after the undergarment has been worn. The panty fastening system 82, therefore, can provide adhesion between the sanitary napkin 20, directly applied to the body prior to panty wearing, and the panty, only after the panty has been pulled up by the user, and more specifically after the contact between the panty fastening system 82 and the panty has already been achieved, contrary to panty fastening systems usually included in disposable absorbent articles, where adhesion with the panty is achieved upon direct contact of a panty fastening adhesive with the panty itself.

FIG. 6 shows a preferred embodiment of the present invention wherein the sanitary napkin 20 of FIG. 1, seen from the garment facing surface 20b comprises onto said garment facing surface 20b of the backsheet 23 a preferred embodiment of the panty fastening system 82. The panty fastening system 82 is preferably comprised in the front region 70 of the sanitary napkin 20, symmetrically centered with respect to the symmetry plane S, and extending longitudinally along less than the length of said front region 70. This is particularly preferred since the front region 70 of the sanitary napkin 20 typically corresponds to the user's mons pubis, where the relative movement between the sanitary napkin 20 and the panty is reduced to a minimum in the wearing conditions, i.e. during the time typically starting after the panty has been pulled up and suitably adjusted with respect to the body, in order to reach a relatively stable condition of minimum internal stress. In other words in this area the panty stays close to the body and typically moves relatively to the body, and to the sanitary napkin 20, much less than in other areas. Attachment of the sanitary napkin 20 to the panty in this area of low relative motion, through the panty fastening system 82 of the present invention, therefore minimises the interaction between the panty and the sanitary napkin during the use, so keeping the increased body fit of the sanitary napkin of the present invention, achieved by means of the preferred tridimensional shape combined with the direct application to the body.

The adhesive means 84 of the panty fastening system 82 define an area of attachment 86, which is the area actually covered by the adhesive means 84.

The actual dimensions of the area of attachment 86 in the panty fastening system 82 is preferably the lowest possible that can provide the desired adhesion to the panty, in order to minimise impact on body fit of the article as explained above and due to possible relative movements between panty and sanitary napkin, while still providing the article with the advantages related to the fastening to the panty, i.e. better capability of staying in its place in contact with the body anatomy during use, and easiness of removal/repositioning. This smallest dimensions of the area of attachment 86 are typically also limited by the highest peel force of the adhesive means 84 onto the panty that is commonly accepted in this type of products, and that is usually determined by easiness of removal of the article from the panty, as can be readily ascertained by the skilled man.

In the preferred embodiment of the present invention illustrated in FIG. 6 and also in the alternative embodiments of FIGS. 8a to 8d, the panty fastening system 82 comprises an area of attachment 86 having e.g. an elliptical shape with the major axis aligned with the symmetry plane S and having a length which is about half the length of the front region 70, and with the minor axis having a length which is about half the width of the sanitary napkin, as measured along the garment facing surface 20b between the longitudinal edges 31 in the front region 70, in correspondence of said minor axis. Different shapes and orientations of the area of attachment 86 are also possible, e.g. ellipses with the major axis oriented transversely with respect to the sanitary napkin, circles, rectangles, or others.

The adhesive means 84 of the panty fastening system 82 of the present invention has a time delayed activation such that the activation of the adhesive means 84 is achieved after the panty has been worn, i.e., typically after the panty has been first pulled up by the user, has subsequently contacted the sanitary napkin 20, and has eventually reached a stable condition of minimum internal stress. During pull up the panty is in fact typically stretched by the user and therefore is deformed and has higher internal stresses due to this stretching. This is the condition in which initial contact between the panty and the panty fastening system 82 of the sanitary napkin 20 is usually achieved, and if an at least partial adhesion between the sanitary napkin 20 and the panty occurred at this stage, successive adjustments and movements of the panty to reach its stable condition of minimum internal stress and stretching/deformation would be reflected on the sanitary napkin 20 by causing the napkin itself to move from its preferred body fitting position achieved through direct application to the body, combined with its preferred tridimensional shape. A time delayed activation of the adhesive means 84 of the panty fastening system 82 of the present invention prevents this from occurring, since adhesion between the sanitary napkin 20 and the panty is achieved only after the initial adjustment of the panty on the user's body has already been made, and the higher initial internal stresses and stretching/deformations of the panty have been released.

Any adhesive means known in the art which is capable of providing said time delayed activation can be comprised in the panty fastening adhesive system 82 of the present invention. For example, adhesive means that are not adhesive by simple contact, or which however develop by simple contact a very low adhesiveness level that does not cause the above described drawbacks, but which can develop the actually desired adhesiveness level by activation by means of pressure, i.e., after the adhesive means and the layer onto which it is intended to be adhered are pressed together, can be incorporated in the panty fastening system 82 of the present invention. Examples of such pressure activated adhesive means are known in the art, and comprise systems where an adhesive either has to flow past a perforated cover or scrim in order to be put in contact with the layer, namely the undergarment, onto which it has to adhere, or where the undergarment has to deform between e.g. standoffs in order to contact the adhesive, such as those described in patents US 5736470, US 5453296, US 4959265. Alternatively, and preferably, deformable structures comprising the adhesive, and in which the adhesive can be put in contact with the layer onto which it is intended to be adhered by means of pressure deforming the structure can be also comprised as adhesive means 84 in the panty fastening means 82 of the present invention, such as for example those described in patents US 4061820, US 5141790, US 5795636, US 5514122, US 4556595. Even more preferred deformable structures comprising a pressure activated adhesive means are those described in patent US 5662758, and in PCT applications WO 97/25268, and WO 97/25256.

The above mentioned pressure activated adhesive means having a time delayed activation can be actually activated by the user herself, after the undergarment has been worn and adjusted on the body, e.g. by exerting a pressure onto the external surface of the panty corresponding to the area 86 of the panty fastening system 82 covered by the adhesive means 84 in order to achieve the attachment between the sanitary napkin 20 and the undergarment. Alternatively, the activation can also be achieved by pressure naturally exerted during the wearing time of the sanitary napkin, after panty pull up and adjustment, e.g. by pressure exerted by tight clothing, e.g. jeans, or by particular wearing conditions, when for example the user rides a bicycle.

According to an alternative embodiment of the present invention the adhesive means 84 having a time delayed activation can also comprise an adhesive composition applied as usual, e.g. in a layer, to a panty fastening system, wherein the adhesive composition has a specifically tailored formulation such that it is not adhesive at room temperature, but is capable of developing pressure sensitive adhesiveness when heated during the use by the users body heat. Of course in this case the activation is not positively achieved by the user, but typically occurs during the wearing time, of course after panty pull up and adjustment, once the adhesive composition has reached the activation temperature by being heated by the users body.

The panty fastening system 82 of the present invention can simply comprise a suitable adhesive means 84 having the time delayed activation, applied onto the garment facing surface 20b of the backsheet 23 of the sanitary napkin 20. However, in the preferred embodiments of the present invention illustrated in FIGS. 6 and 8a to 8d the adhesive means 84 is comprised onto a substrate 88 which is joined to the garment facing surface 20b of the of the backsheet 23 of the sanitary napkin 20 at attached portions 90a, and is not joined to the backsheet 23 at unattached portions 90b, such that the substrate 88 can be separated from the backsheet 23 at the unattached portions 90b during the use of the sanitary napkin. This preferred partial attachment of the substrate 88 comprising the panty fastening system 82 to the backsheet 23 of the sanitary napkin 20 provides for an even increased independence between the sanitary napkin 20 and the undergarment as far as relative movements during the wearing time are concerned. This in fact means for example that major movements of the panty induced during particularly critical wearing conditions, e.g. with the user running, or squatting, or cycling, are only to a minimum extent transferred to the sanitary napkin 20 owing to the preferred partial attachment of the substrate 88 to the backsheet 23, which provides decoupling between relative movements of the sanitary napkin 20 and of the panty. This works also the other way around, when for example further adjustments of the sanitary napkin 20 to the body shape are not prevented by a too tight attachment of the sanitary napkin 20 to the panty. At the same time, the panty fastening system 82 provides an effective attachment between sanitary napkin 20 and undergarment which is beneficial for the reasons explained hereinabove in the Background of the Invention.

Preferably, as it is illustrated in FIG. 6 and in FIG. 8a, the substrate has longitudinal side edges 92 which are substantially parallel to the symmetry plane S, and the substrate is joined to the backsheet 23 along said longitudinal side edges 92, which in turn constitute the attached portions 90b of the substrate 88. Alternatively, as illustrated in FIG. 8b, the substrate 88 is joined to the backsheet 23 along the longitudinal side edges 92 and also along the front transverse edge 94a, while in FIG. 8c the substrate 88 is joined to the backsheet 23 along the longitudinal side edges 92, and also along both the front and rear transverse edges 94a and 94b. FIG. 8d shows a further embodiment of the present invention in which the substrate 88 is joined to the backsheet 23 only at its four corners 97. In all these embodiments the substrate 88 of the panty fastening system 82 has a substantially rectangular shape, but different shapes are also possible, and necessary variations as far as the positioning of the attached and unattached portions 90a and 90b is concerned can be readily made by the man skilled in the art.

In the particularly preferred embodiment of the present invention illustrated in FIG. 6 the substrate 88 further comprises along its longitudinal side edges 92 two longitudinal pleats 96 along which it is joined to the backsheet 23. This is more clearly shown in the sectional view of FIG. 5a, and the pleats 96 provide the substrate 88 with an ever increased decoupling capability with respect to the sanitary napkin 20, more specifically allowing relative movements between the substrate 88 of the panty fastening system 82 and the sanitary napkin 20 along the transverse direction and also along directions locally perpendicular to the substrate 88, e.g. comprised in the symmetry plane S. Alternatively, the substrate 88 can be joined to the backsheet 23 through portions of a stretchable, or also elastically stretchable material, or the substrate itself 88 can be constituted by a stretchable, or elastically stretchable material.

According to the embodiment of the present invention illustrated in FIG. 6, and as also visible in FIGS. 2 and 5a, the adhesive means 84 of the panty fastening adhesive system 82 in the sanitary napkin 20 comprises distinct recessed areas 98, having for example a circular shape, and a conventional pressure sensitive adhesive 100 is comprised at the bottom of the recessed areas 98. The recessed areas 98 create standoffs between them in the substrate 88, and the activation of the adhesive means 84 is achieved by pressure by deforming the garment in order to have it contact the adhesive 100 between the standoffs.

The recessed areas can be created e.g. in the substrate 88 by deforming it, with the adhesive composition 100 then applied at the bottom of those recessed areas 98 only, or, alternatively, as illustrated in FIGS. 2 and 5a, the adhesive composition 100 can be uniformly applied to the substrate 88, and then a further layer 102 having e.g. circular apertures is applied onto this uniform adhesive layer, wherein the recessed areas 98 are created by the thickness of this further layer 102. The adhesive composition 100 is exposed at the bottom of the recessed areas 98 through the apertures of the layer 102, and the activation of the adhesive means 84 in the panty fastening system 82 is achieved in this embodiment as already explained above.

Structures functionally equivalent to those described above that can be preferably incorporated as adhesive means 84 having a time delayed activation (namely activation by means of pressure) in the panty fastening adhesive 82 of the present invention are those descried in the already mentioned patent US 5662758, and in PCT applications WO 97/25268, and WO 97/25256. According to these disclosures, the structures broadly comprise a three dimensional layer with recessed areas therein and an adhesive composition located at the bottom of said recessed areas. Activation of the adhesive is done by pressure, wherein the three dimensional structure of the layer is deformed into a substantially two dimensional structure in order to expose or release the adhesive and bring it in contact with the surface where adhesion has to be achieved.

It is not necessary that the panty fastening system according to the present invention be covered with a removable release paper or film as it is typical in known disposable absorbent articles. However, in order to prevent the adhesive means from drying out or adhering to another surface other than the panty by being accidentally activated a removable release liner or more simply a separation layer can be advantageously incorporated in the disposable absorbent article of the present invention, depending on the adhesive means comprised in the panty fastening system. Any suitable material may be used, such as preferably a film.

In a further alternative embodiment of the present invention the tridimensional disposable absorbent article can comprise a body adhesive on its body facing surface in order to be adhered directly to the wearer's body, preferably with no need of a panty fastening adhesive.

The tridimensional absorbent articles of the present invention, particularly the sanitary napkin 20, have a length that preferably ranges among the typical values commonly used for different sizes of said sanitary articles intended for substantially external disposition adjacent to the body of the wearer. Particularly, the central and rear portions 42 and 44 of the absorbent core 24 do not have preferably a length which is smaller than the total maximum length of the labia majora of an average user.

The tridimensional absorbent article of the present invention may further comprise an odour-control material for controlling unpleasant odours associated with absorbed body fluids.

Any known odour-control agent or any combination thereof that can be suitably included in a disposable absorbent article, including other materials such as binders and/or substrates, can be comprised in the absorbent article of the present invention as the odour-control material.

The odour-control material can be incorporated into the absorbent article by methods known in the art, for example layered on or into the absorbent core or mixed within the absorbent core.

In an alternate embodiment of the present invention, the absorbent article may have two flaps (not shown), each of which is adjacent to and extends laterally from the respective side edge of the absorbent core. The flaps are configured to drape over the edges of the wearers panties in the crotch region so that the flaps are disposed between the edges of the wearers panties and the wearers thighs. The flaps help serve to prevent soiling of the wearers body and panties by menstrual fluid, preferably by forming a double wall barrier along the edges of the panty. The flaps may be also provided with attachment means on their garment facing surface so that the flaps can be folded back under the panty and attached to the garment facing side of the panty.

The flaps may be constructed of various materials including materials used for the topsheet 22, backsheet 23, combinations thereof, and may be a laminate having tissue in the centre. Further, the flaps may be a separate element attached to the main body of the tridimensional absorbent article or can comprise extensions of the topsheet 22 and/or backsheet 23. It is recommended, however, that the flaps have a liquid impervious backsheet to prevent body fluids which reach the flaps from soiling the edges of the wearers panties.

Preferred flaps that are suitable or adaptable to the tridimensional absorbent article of the present invention are disclosed in U.S. Pat. No. 4,687,478 issued to Van Tilburg on Aug. 18, 1987; U.S. Pat. No. 4,589,876 issued to Van Tilburg on May 20, 1986; and U.S. Pat No. 4,608,047 issued to Mattingly on Aug. 26, 1986.

Optionally, but preferably over the above mentioned flaps, the tridimensional absorbent article may comprise components that naturally wrap the sides of a wearers panties. Sanitary napkins having components that naturally wrap the sides of a wearer's panties suitable for use with the tridimensional absorbent article of the present invention are disclosed in U.S. Patent Application Serial No. 08/096,121 entitled "Absorbent Article having Panty Covering Components that Naturally Wrap the Sides of Panties", filed July 22, 1993, in the names of Lavash, et al and U.S. Patent Application Serial No. 08/277733 entitled "Absorbent Articles Having Undergarment Covering Components with Zones of Extensibility", filed July 20, 1994, in the names of Weinberger, et al.

In further alternate embodiments of the present invention the absorbent article can also comprise additional elements, such as an acquisition layer or a secondary topsheet positioned between the topsheet 22 and the absorbent core 24 or, alternatively, in any other suitable position.

Although the disposable absorbent article of the present invention has been described with reference to a sanitary napkin, it can be used beneficially in the context of other disposable absorbent articles such as panty liners and incontinence articles. The disposable absorbent article may thus also have all those features and parts which are typical for products in the context of their intended use.

## Claims

1. A tridimensional disposable absorbent article for wearing against a user's body, intended for direct application to the user's body before an undergarment is worn, said tridimensional disposable absorbent article having a tridimensional shape prior to use, said tridimensional disposable absorbent article having a body facing surface and a garment facing surface, a front region, a rear region, a central region comprised between said front region and said rear region, and a longitudinal symmetry plane,
said tridimensional disposable absorbent article comprising a liquid pervious topsheet, a liquid impervious backsheet joined to said topsheet, and an absorbent core intermediate said topsheet and said backsheet,
said tridimensional disposable absorbent article further comprising on said garment facing surface of said backsheet a panty fastening system for attachment of said article to an undergarment during the use of said article,
said tridimensional disposable absorbent article characterized in that
said panty fastening system comprises an adhesive means having a time delayed activation, such that said activation is achieved after said undergarment has been worn.

2. A tridimensional disposable absorbent article according to claim 1, characterized in that said adhesive means of said panty fastening system is activatable by the user by means of pressure.

3. A tridimensional disposable absorbent article according to claim 1, characterized in that said adhesive means of said panty fastening system is an adhesive activatable by means of the body temperature.

4. A tridimensional disposable absorbent article according to any preceding claim, characterized in that said panty fastening system is comprised in said front region of said disposable absorbent article.

5. A tridimensional disposable absorbent article according to any preceding claim, characterized in that said panty fastening system comprises a substrate joined to said garment facing surface of said backsheet, wherein said substrate comprises said adhesive means, and further in that said substrate is joined to said backsheet at attached portions, and is not joined to said backsheet at unattached portions, whereby said substrate can be separated from said backsheet at said unattached portions, to provide decoupling between movements of said article and of said undergarment.

6. A tridimensional disposable absorbent article according to claim 5, characterized in that said substrate has longitudinal side edges, wherein said substrate is joined to said backsheet along said longitudinal side edges.

7. A tridimensional disposable absorbent article according to claim 6, characterized in that said substrate comprises longitudinal pleats joining said substrate to said backsheet at said longitudinal side edges.

8. A tridimensional disposable absorbent article according to any preceding claim, characterized in that said panty fastening system comprises distinct recessed areas, wherein a pressure sensitive adhesive is comprised in said distinct recessed areas.

9. Use of a tridimensional disposable absorbent article according to claim 1, characterized in that said activation is achieved by pressure induced by the wearing conditions.
